(19) Europäisches Patentamt
European Patent Office
Office européen des brevets

(11) Veröffentlichungsnummer: **0 400 344 B1**

(12) **EUROPÄISCHE PATENTSCHRIFT**

(45) Veröffentlichungstag der Patentschrift: **15.12.93**

(51) Int. Cl.[5]: **C07D 213/30**, C07D 213/89, C07D 405/06, C07D 409/06, A01N 43/10

(21) Anmeldenummer: **90108294.1**

(22) Anmeldetag: **02.05.90**

Die Akte enthält technische Angaben, die nach dem Eingang der Anmeldung eingereicht wurden und die nicht in dieser Patentschrift enthalten sind.

# (54) Pyridinderivate und ihre Verwendung als Fungizide.

(30) Priorität: **05.05.89 DE 3914820**

(43) Veröffentlichungstag der Anmeldung: **05.12.90 Patentblatt 90/49**

(45) Bekanntmachung des Hinweises auf die Patenterteilung: **15.12.93 Patentblatt 93/50**

(84) Benannte Vertragsstaaten: **AT BE CH DE DK ES FR GB GR IT LI NL SE**

(56) Entgegenhaltungen:
**EP-A- 0 302 365**
**EP-A- 0 302 366**
**EP-A- 0 311 907**

(73) Patentinhaber: **BASF Aktiengesellschaft**
**Carl-Bosch-Strasse 38**
**D-67063 Ludwigshafen(DE)**

(72) Erfinder: **Zipperer, Eberhard, Dr.**
**Am Herrgottsacker 6**
**D-6716 Dirmstein(DE)**
Erfinder: **Sauter, Hubert, Dr.**
**Neckarpromenade 20**
**D-6800 Mannheim 1(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

EP 0 400 344 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Pyridinderivate, deren N-Oxide und Salze, Verfahren zu ihrer Herstellung und ihre Verwendung zur Bekämpfung von Pilzen.

Die neuen Pyridinderivate haben die Formel

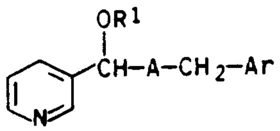

I

in der

| | |
|---|---|
| $R^1$ | für Wasserstoff, $C_1$-$C_6$-Alkyl, $C_3$-$C_6$-Alkenyl, $C_3$-$C_6$-Alkinyl oder einen Acylrest $COR^2$ steht; |
| $R^2$ | für $C_1$-$C_6$-Alkyl, $C_3$-$C_6$-Alkenyl, $C_3$-$C_6$-Alkinyl, $C_1$-$C_6$-Halogenalkyl, Napthyl oder Phenyl, die gegebenenfalls ein bis drei Substituenten, ausgewählt aus der Gruppe bestehend aus $C_1$-$C_4$-Alkyl, $C_1$-$C_4$-Alkoxy, $C_1$-$C_4$-Halogenalkyl, Halogen, Cyano und Nitro, tragen, steht; |
| A | für die Gruppen |

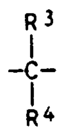 , 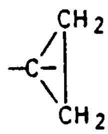 oder 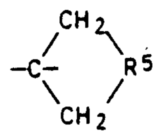

steht;

| | |
|---|---|
| $R^3$, $R^4$ | unabhängig voneinander für $C_1$-$C_6$-Alkyl stehen, mit der Maßgabe, daß nicht $R^3$ und $R^4$ gleichzeitig Methyl bedeuten; |
| $R^5$ | für eine der Gruppen $(CH_2)_n$, $CH_2OCH_2$, $CH_2S(O)_mCH_2$, $CH_2CH_2O$, $CH_2CH_2$ $S(O)_m$ steht, wobei |
| n | eine ganze Zahl von 1 bis 5 und |
| m | eine ganze Zahl von 0 bis 2 bedeutet; |
| Ar | für Phenyl, Naphthyl oder Indanyl steht, die gegebenenfalls 1-5 Substituenten aus der Gruppe $C_1$-$C_6$-Alkyl, Phenyl, Halogen, $C_1$-$C_6$-Halogenalkyl, $C_1$-$C_6$-Alkoxy, $C_1$-$C_6$-Halogenalkoxy, $C_1$-$C_6$-Alkoxyiminocarbyl, Phenoxy, Halogenphenoxy, Benzyloxy tragen, und die N-Oxide und die pflanzenverträglichen Säureadditionssalze der Pyridinverbindungen. |
| $R^1$ | bedeutet beispielsweise Wasserstoff, Methyl, Ethyl, n-Propyl, Isopropyl, n-Butyl, Isobutyl, tert.-Butyl, n-Pentyl, iso-Pentyl, n-Hexyl; Allyl, 2-Methylallyl, 3-Methylallyl, 3,3-Dimethylallyl; Propargyl. |
| $R^2$ | bedeutet beispielsweise Methyl, Ethyl, n-Propyl, Isopropyl, n-Butyl, Isobutyl, tert.-Butyl, n-Pentyl, Isopentenyl, neo-Pentyl, n-Hexyl; Trifluormethyl, Chlormethyl, Dichlormethyl, Trichlormethyl, Brommethyl, 2-Bromethyl, 2-Chlorethyl, 3-Brompropyl, 4-Brombutyl; Naphthyl oder Phenyl, die gegebenenfalls ein bis drei Substituenten, ausgewählt aus der Gruppe bestehend aus $C_1$-$C_4$-Alkyl, $C_1$-$C_4$-Alkoxy, $C_1$-$C_4$-Halogenalkyl, Halogen, Cyano und Nitro, tragen, wie Naphthyl, Phenyl, Mono-, Di- oder Trimethylphenyl, 4-tert.-Butylphenyl, Mono-, Di- oder Trimethoxyphenyl, Trifluormethylphenyl, Fluorphenyl, Mono-, Di- oder Trichlorphenyl, Mono- oder Dinitrophenyl, Cyanphenyl. |
| $R^3$, $R^4$ | bedeuten unabhängig voneinander beispielsweise Methyl, Ethyl, n-Propyl, Isopropyl, n-Butyl, Isobutyl, tert.-Butyl, n-Pentyl, Isopentyl, n-Hexyl. |
| $R^5$ | bedeutet beispielsweise Methylen, Ethylen, 1,3-Propylen₁ 1,4-Butylen, 1,5-Pentylen, Methylenoxymethylen, Ethylenoxy, Methylenthiomethylen, Methylensulfoxymethylen, Methylensulfonylmethylen, Ethylenthio, Ethylensulfinyl, Ethylensulfonyl. |
| Ar | bedeutet beispielsweise Phenyl, Naphthyl, Indanyl, Mono-, Di-, Tri- oder Tetramethylphenyl, Ethylphenyl, Propylphenyl, Iso-propylphenyl, Butylphenyl, tert.-Butylphenyl, Biphenyl, Methylnaphthyl, Mono- oder Difluorphenyl, Methyl-fluorphenyl, Tri-, Tetra- oder Pentafluorphenyl, Fluor-chlorphenyl, Methyl-chlorphenyl, Mono-, Di-, Tri-, Tetra- oder Pentachlorphenyl, Bromphenyl, Fluor-bromphenyl, Mono- oder Bis-trifluormethylphenyl, Mono-, Di- oder Trimethoxyphenyl, Ethoxyphenyl, Trifluormethoxyphenyl, Tetrafluorethoxyphenyl, Methoxyiminocarbylp- |

henyl, Ethoxyiminocarbylphenyl, Phenoxyphenyl, Fluorphenoxyphenyl, Chlorphenoxyphenyl, Chlorphenoxy-chlorphenyl, Benzyloxyphenyl.

Bevorzugt werden Verbindungen der Formel 1, worin

$R^1$ Wasserstoff, Methyl, Ethyl, tert.-Butyl, Allyl, Propargyl oder $COR^2$,

$R^2$ $C_1$-$C_6$-Alkyl, $C_1$-$C_6$-Halogenalkyl oder gegebenenfalls substituiertes Phenyl,

A eine der Gruppen

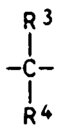 , 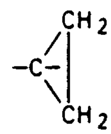 oder 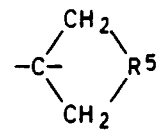 ,

$R^3$, $R^4$ unabhängig voneinander Methyl, Ethyl, n-Propyl, n-Butyl, n-Pentyl, n-Hexyl,

$R^5$ eine Ethylen- oder eine 1,3-Propylengruppe bedeuten und worin Ar die in Anspruch 1 genannten Bedeutungen hat.

Die Erfindung betrifft auch die N-Oxide und die pflanzenverträglichen Säureadditionssalze der neuen Pyridinderivate. Pflanzenphysiologisch verträgliche Salze der Pyridine sind z.B. Salze mit anorganischen Mineralsäuren wie Hydrochloride, Hydrobromide, Sulfate, Nitrate, Phosphate; Salze mit Alkylcarbonsäuren wie Formiate, Acetate, Propionate, 2-Ethylhexanoate, Oxalate; Salze mit Arylsulfonsäuren wie z.B. Benzolsulfonate, Toluolsulfonate, Dodecylbenzolsulfonate.

Die Verbindungen der Formel 1 können gegebenenfalls zwei Asymmetrie-Zentren aufweisen und daher in zwei diastereomeren Formen vorliegen, welche durch bekannte Verfahren, beispielsweise durch Chromatographie oder Kristallisation, getrennt werden können. Die vorliegende Erfindung umfaßt sowohl die einzelnen Diastereomeren als auch deren Mischungen und diese Verbindungen enthaltende Fungizide.

Die Verbindungen der Formel I haben eine gute fungizide Wirkung, die besser ist als die Wirkung üblicher bekannter Fungizide.

Es ist bekannt, Pyridylcarbinole, z.B. das 3-Phenyl-2,2-dimethyl-1-(3-pyridyl)-propanol-1, - bekannt aus der europäischen Patentanmeldung EP 302 365 - als Fungizide zu verwenden. Ihre fungizide Wirkung ist jedoch in bestimmten Indikationsbereichen, insbesondere bei niedrigen Aufwandmengen, nicht immer ganz befriedigend.

Das Verfahren zur Herstellung der neuen Verbindungen der Formel 1 ist dadurch gekennzeichnet, daß man einen Aldehyd der Formel 2,

$Ar-CH_2-A-CHO$ 2

in der Ar und A die in Anspruch 1 genannten Bedeutungen haben, umsetzt mit einer Organometallverbindung der Formel 3,

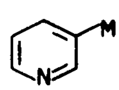

3

in der M für Lithium oder einen der Reste MgCl, MgBr oder MgI steht, und den dabei gebildeten Pyridylalkohol der Formel 1, in der A und Ar die in Anspruch 1 genannten Bedeutungen haben und $R^1$ für Wasserstoff steht, gegebenenfalls mit einer Verbindung der Formel 4 alkyliert oder mit einer Verbindung der Formel 5 acyliert,

$R^1-X$

4

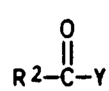

5

in denen $R^1$ und $R^2$ die in Anspruch 1 genannten Bedeutungen mit Ausnahme von Wasserstoff haben und X bzw. Y für eine nucleofuge Gruppe stehen.

X bedeutet beispielsweise Chlor, Brom, Jod, $OSO_2CH_3$, $OSO_2C_6H_5$, $OSO_2$-$C_6H_4$-$CH_3$ (p).

Y bedeutet beispielsweise Chlor, Brom, Jod,

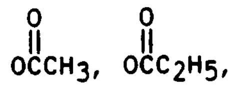

1-Imidazolyl.

Beispielsweise wird bei dieser Umsetzung so vorgegangen, daß ein Gemisch aus Pyridylalkohol der Formel 1, in der $R^1$ Wasserstoff bedeutet, zusammen mit mindestens der äquimolaren Menge einer Hilfsbase, mit oder ohne ein inertes, organisches Lösungsmittel wie Diethylether, Tetrahydrofuran, Dioxan, Dimethylformamid, Dimethylsulfoxid, Acetonitril, Toluol, Xylol vorgelegt wird und danach das Alkylierungs- oder Acylierungsmittel zudosiert wird.

Die Reaktionstemperaturen betragen beispielsweise 0 bis 120 °C, vorzugsweise 20 bis 80 °C.

Als Hilfsbasen können anorganische und organische Säurebindungsmittel verwendet werden.

Beispiele für anorganische Basen sind Natriumhydrid, Natriumamid, Natriumhydroxid, Natriumcarbonat, Kaliumcarbonat, Kaliumhydroxid und andere.

Beispiele für organische Basen sind Amine, insbesondere tertiäre Amine wie Triethylamin, Ethyldiisopropylamin, Pyridin und Alkoholate wie Natriummethylat, Natriumethylat, Kalium-tert.-butylat.

In einer zuweilen vorteilhaften Variante dieses Verfahrens werden die Umsetzungen der Pyridylalkohole der Formel 1 mit Alkylierungsmitteln der Formel 4 in einem Zweiphasensystem, bestehend aus wässriger Natronlauge und einem organischen Lösungsmittel, vorzugsweise Toluol oder Dichlormethan, unter Zusatz eines Phasentransferkatalysators, z.B. Tetra-n-butylammoniumchlorid,Benzyltriethylammoniumchlorid, Methyltrioctylammoniumchlorid vorgenommen.

Die neuen Pyridylalkohole der Formel 1, in der $R^1$ für Wasserstoff steht, können beispielsweise hergestellt werden, indem man ein metalliertes Pyridin der Formel 3, in der M für Lithium oder einen der Reste MgCl, MgBr oder MgI steht mit einem Aldehyd der Formel 2, in der Ar und A die in Anspruch 1 genannten Bedeutungen haben, z.B. in Gegenwart eines geeigneten Lösungsmittels umsetzt.

Dabei ist es von Vorteil, die Organometallverbindung der Formel 3 in einem inerten Lösungsmittel, vorzugsweise einem Ether wie Diethylether oder Tetrahydrofuran, bei Temperaturen von -100 bis +20 °C, bevorzugt von -70 bis 0 °C, vorzulegen und den Aldehyd der Formel 2, gegebenenfalls gelöst in einem Verdünnungsmittel, zuzutropfen.

Die metallierten Pyridine der Formel 3 sind bekannt und können aus den entsprechenden 3-Halogenpyridinen hergestellt werden (vgl. z.B. N. Furukawa et. al., Tetrahedron Letters 1987, 5845 für M = MgBr; A. Fischer und M.W. Morgan, J. Organomet. Chem. 136 (1977) 323 für M = Li.).

Die Aldehyde der Formel 2 sind teilweise bekannt und können, nach an sich bekannten Verfahren, dadurch hergestellt werden, daß man Aldehyde der Formel (6), in der A die in Anspruch 1 genannten Bedeutungen hat mit einem Benzylhalogenid der Formel (7) alkyliert:

$$\underset{(6)}{H\text{-}A\text{-}CHO} \quad + \quad \underset{(7)}{Ar\text{-}CH_2\text{-}Hal} \longrightarrow \underset{(2)}{Ar\text{-}CH_2\text{-}A\text{-}CHO}$$

(Hal = Cl,Br)

Es ist bekannt, daß die direkte $\alpha$-Alkylierung von Aldehyden in der Regel nur mit schlechten Ausbeuten verläuft; gleiches gilt auch für die von den Aldehyden abgeleiteten Enamine (vgl. z.B. G. Opitz et. al., Liebigs Ann. Chem 649 (1961) 36). Bessere Ergebnisse erhält man, wenn man die Aldehyde der Formel 6 zunächst in N-metallierte Imine überführt, die anschließend mit Benzylhalogeniden alkyliert werden (vgl. H. Normant et. al., Bull. Soc. Chim. Fr. 1970, 3976). Eine präparativ einfachere Methode ist die Phasentransfer-katalysierte $\alpha$-Alkylierung von Aldehyden. Dieses Verfahren kann als fest/flüssig-Variante in einem Zweiphasensystem, bestehend aus festem Natriumhydroxid und einem lipophilen, organischen Lösungsmittel (vgl. V.G. Poruhit und R. Subramanian, Chem. Ind. (London) 1978, 731) oder als flüssig/flüssig-Variante in einem

Zweiphasensystem, bestehend aus konzentrierter Natronlauge und einem lipophilen, organischen Lösungsmittel (vgl. H.K. Dietl und K.C. Brannock, Tetrahedron Lett. 1973, 1273; E. Buschmann und B. Zeeh, Liebigs Ann. Chem. 1979, 1585) durchgeführt werden.

Ein weiteres Verfahren zur Herstellung von Aldehyden der Formel (2) ist dadurch gekennzeichnet, daß man Nitrile der Formel 8 mit Benzylhalogeniden der Formel 7 alkyliert und die Reaktionsprodukte der Formel 9 mit einem geeigneten Reduktionsmittel in die Aldehyde 2 überführt:

$$\underset{(8)}{H{-}A{-}CN} + \underset{(7)}{Ar{-}CH_2{-}Hal} \longrightarrow \underset{(9)}{Ar{-}CH_2{-}A{-}CN} \longrightarrow \underset{(2)}{Ar{-}CH_2{-}A{-}CHO}$$

Die α-Alkylierung von Nitrilen und die Reduktion von Nitrilen zu Aldehyden sind bekannte Reaktionen (vgl. z.B. D.S. Watt et. al, Org. React. 31 (1984) 1 ff.; M. Rabinovitz in Z. Rappoport (Hrsg.) "The Chemistry of the Cyano Group", S. 307 ff, Interscience, New York 1970; J. Malek und M. Cerny, Synthesis 1972, 217; N.M. Yoon und Y.S. Gyoung, J. Org. Chem. 50 (1985) 2443).

Die als Ausgangsstoffe eingesetzten Aldehyde (6), Nitrile (8) und Benzylhalogenide (7) sind bekannt und größtenteils im Handel erhältlich.

Die folgenden Beispiele erläutern die Herstellung der neuen Pyridinderivate.

Beispiel 1

2-(4-Chlorbenzyl)-2-ethyl-1-(3-pyridyl)-butyl-1-acetat
(Verbindung Nr. 611)

Zur Lösung von 12,1 g (0,04 mol) 2-(4-Chlorbenzyl)-2-ethyl-1-(3-pyridyl)butanol-1 in 50 ml Pyridin tropft man bei Raumtemperatur 10,2 g (0,10 mol) Essigsäureanhydrid und erwärmt anschließend 8 h auf 80°C. Die Reaktionsmischung wird eingeengt, der Rückstand in 200 ml Dichlormethan aufgenommen und mit gesättigter $NaHCO_3$-Lösung, dann mit Wasser gewaschen. Nach Trocknen und Einengen der organischen Phase wird das verbleibende schwarze Öl an Kieselgel (Laufmittel: Cyclohexan/Ethylacetat 5:1) gereinigt. Man erhält 11,2 g (81 % d. Th.) der Titelverbindung als zähes, gelbes Öl.

$^1$H-NMR ($CDCl_3$): $\delta$ = 8.60(2-Hpy), 8.52(6-Hpy), 7.65(4-Hpy), 7.30(5-Hpy), 7.25(2Hm), 7.05(2Ho), 5.62-(CHOAc), 2.68($CH_2Ar$), 2.08($COCH_3$), 1.45($CH_2$), 1.30($CH_2$), 0.95($CH_3$), 0.83($CH_3$).

Beispiel 2

2-(4-Chlorbenzyl)-2-ethyl-1-(3-pyridyl)-butanol-1
(Verbindung Nr. 113)

Zu einem Gemisch aus 82 ml einer 1,6 M n-Butyllithium-Lösung in n-Hexan (0,13 mol) tropft man unter Stickstoffatmosphäre bei -70°C die Lösung von 15,8 g 3-Brompyridin (0,10 mol) in 50 ml Diethylether. Man rührt 10 min bei -70°C nach und tropft dann die Lösung von 24,7 g 2-(4-Chlorbenzyl)-2-ethyl-butanal (0,11 mol) in 50 ml Diethylether zu. Nach 1 h bei -70°C läßt man langsam auf Raumtemperatur erwärmen und hydrolysiert mit gesättigter, wässriger Ammoniumchloridlösung. Die Mischung wird mit verd. Salzsäure sauer gestellt, die wässrige Phase wird abgetrennt und mit Ether gewaschen, danach mit konz. Ammoniaklösung unter Kühlen auf pH 10 bis 11 gebracht. Man extrahiert dreimal mit Dichlormethan, trocknet die organische Phase über $Na_2SO_4$ und engt ein. Das feste Rohprodukt wird aus Essigester/Hexan (1:1) umkristallisiert. 19,2 g (63 % d. Th.) farblose Kristalle vom Schmp. 128-129°C.

Herstellung der Ausgangsverbindung

2-(4-Chlorbenzyl)-2-ethyl-butanal

300 ml Toluol, 400 ml 30 proz. (Gew.-%) Natronlauge und 5,0 g Tetra-n-butylammoniumjodid werden unter Rühren auf 80°C erwärmt. Dazu tropft man langsam ein Gemisch aus 110 g (1,10 mol) 2-Ethylbutanal und 161 g (1,00 mol) 4-Chlorbenzylchlorid. Man rührt noch 4 h bei 80°C und 14 h bei Raumtemperatur nach, versetzt mit 1000 ml Toluol und trennt die Phasen. Die organische Phase wird mit Wasser

gewaschen, über $Na_2SO_4$ getrocknet und eingeengt: Nach fraktionierender Destillation erhält man 123 g (54 % d. Th.) 2-(4-Chlorbenzyl)-2-ethyl-butanal als farblose Flüssigkeit vom Sdp. 146-148 °C bei 1 mbar.

Auf entsprechende Weise wie in den Beispielen 1 und 2 können auch folgende Verbindungen hergestellt werden:

Tabelle 1

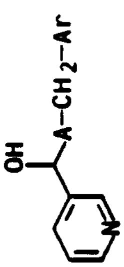

| Bsp.Nr. | A | Ar | Phys. Daten |
|---|---|---|---|
| 1 | $C(CH_3)C_2H_5$ | Phenyl | |
| 2 | $C(CH_3)C_2H_5$ | 2-Methylphenyl | |
| 3 | $C(CH_3)C_2H_5$ | 3-Methylphenyl | |
| 4 | $C(CH_3)C_2H_5$ | 4-Methylphenyl | |
| 5 | $C(CH_3)C_2H_5$ | 2,3-Dimethylphenyl | |
| 6 | $C(CH_3)C_2H_5$ | 2,4-Dimethylphenyl | |
| 7 | $C(CH_3)C_2H_5$ | 2,5-Dimethylphenyl | |
| 8 | $C(CH_3)C_2H_5$ | 2,6-Dimethylphenyl | |
| 9 | $C(CH_3)C_2H_5$ | 3,4-Dimethylphenyl | |
| 10 | $C(CH_3)C_2H_5$ | 3,5-Dimethylphenyl | |
| 11 | $C(CH_3)C_2H_5$ | 2,4,5-Trimethylphenyl | |
| 12 | $C(CH_3)C_2H_5$ | 2,4,6-Trimethylphenyl | |
| 13 | $C(CH_3)C_2H_5$ | 2,3,5,6-Tetramethylphenyl | |
| 14 | $C(CH_3)C_2H_5$ | 4-Ethylphenyl | |
| 15 | $C(CH_3)C_2H_5$ | 4-Isopropylphenyl | |
| 16 | $C(CH_3)C_2H_5$ | 4-tert.Butylphenyl | |
| 17 | $C(CH_3)C_2H_5$ | 5-Indanyl | |
| 18 | $C(CH_3)C_2H_5$ | 1-Naphthyl | |
| 19 | $C(CH_3)C_2H_5$ | 2-Naphthyl | |

Tabelle 1 (Fortsetzung)

| Bsp.Nr. | A | Ar | Phys. Daten |
|---|---|---|---|
| 20 | $C(CH_3)C_2H_5$ | 2-Methyl-1-naphthyl | |
| 21 | $C(CH_3)C_2H_5$ | 4-Biphenyl | |
| 22 | $C(CH_3)C_2H_5$ | 2-Fluorphenyl | |
| 23 | $C(CH_3)C_2H_5$ | 3-Fluorphenyl | |
| 24 | $C(CH_3)C_2H_5$ | 4-Fluorphenyl | |
| 25 | $C(CH_3)C_2H_5$ | 2,3-Difluorphenyl | |
| 26 | $C(CH_3)C_2H_5$ | 2,4-Difluorphenyl | |
| 27 | $C(CH_3)C_2H_5$ | 2,5-Difluorphenyl | |
| 28 | $C(CH_3)C_2H_5$ | 2,6-Difluorphenyl | |
| 29 | $C(CH_3)C_2H_5$ | 3,4-Difluorphenyl | |
| 30 | $C(CH_3)C_2H_5$ | 3,5-Difluorphenyl | |
| 31 | $C(CH_3)C_2H_5$ | 2-Fluor-3-methylphenyl | |
| 32 | $C(CH_3)C_2H_5$ | 2,3,5,6-Tetrafluorphenyl | |
| 33 | $C(CH_3)C_2H_5$ | Pentafluorphenyl | |
| 34 | $C(CH_3)C_2H_5$ | 2-Chlorphenyl | |
| 35 | $C(CH_3)C_2H_5$ | 3-Chlorphenyl | |
| 36 | $C(CH_3)C_2H_5$ | 4-Chlorphenyl | |
| 37 | $C(CH_3)C_2H_5$ | 2,3-Dichlorphenyl | |
| 38 | $C(CH_3)C_2H_5$ | 2,4-Dichlorphenyl | |
| 39 | $C(CH_3)C_2H_5$ | 2,5-Dichlorphenyl | |
| 40 | $C(CH_3)C_2H_5$ | 2,6-Dichlorphenyl | |
| 41 | $C(CH_3)C_2H_5$ | 3,4-Dichlorphenyl | |
| 42 | $C(CH_3)C_2H_5$ | 3,5-Dichlorphenyl | |
| 43 | $C(CH_3)C_2H_5$ | 2,4,5-Trichlorphenyl | |
| 44 | $C(CH_3)C_2H_5$ | 2,4,6-Trichlorphenyl | |

Tabelle 1 (Fortsetzung)

| Bsp.Nr. | A | Ar | Phys. Daten |
|---|---|---|---|
| 45 | $C(CH_3)C_2H_5$ | 2,3,6-Trichlorphenyl | |
| 46 | $C(CH_3)C_2H_5$ | 2-Chlor-5-methylphenyl | |
| 47 | $C(CH_3)C_2H_5$ | 3-Chlor-4-methylphenyl | |
| 48 | $C(CH_3)C_2H_5$ | 2-Chlor-4-fluorphenyl | |
| 49 | $C(CH_3)C_2H_5$ | 2-Chlor-6-fluorphenyl | |
| 50 | $C(CH_3)C_2H_5$ | 3-Chlor-2-fluorphenyl | |
| 51 | $C(CH_3)C_2H_5$ | Pentachlorphenyl | |
| 52 | $C(CH_3)C_2H_5$ | 2-Bromphenyl | |
| 53 | $C(CH_3)C_2H_5$ | 3-Bromphenyl | |
| 54 | $C(CH_3)C_2H_5$ | 4-Bromphenyl | |
| 55 | $C(CH_3)C_2H_5$ | 2-Fluor-4-bromphenyl | |
| 56 | $C(CH_3)C_2H_5$ | 2-Trifluormethylphenyl | |
| 57 | $C(CH_3)C_2H_5$ | 3-Trifluormethylphenyl | |
| 58 | $C(CH_3)C_2H_5$ | 4-Trifluormethylphenyl | |
| 59 | $C(CH_3)C_2H_5$ | 2,4-Bis(trifluormethyl)phenyl | |
| 60 | $C(CH_3)C_2H_5$ | 3,5-Bis(trifluormethyl)phenyl | |
| 61 | $C(CH_3)C_2H_5$ | 2-Methoxyphenyl | |
| 62 | $C(CH_3)C_2H_5$ | 3-Methoxyphenyl | |
| 63 | $C(CH_3)C_2H_5$ | 4-Methoxyphenyl | |
| 64 | $C(CH_3)C_2H_5$ | 3,4-Dimethoxyphenyl | |
| 65 | $C(CH_3)C_2H_5$ | 3,4,5-Trimethoxyphenyl | |
| 66 | $C(CH_3)C_2H_5$ | 4-Ethoxyphenyl | |
| 67 | $C(CH_3)C_2H_5$ | 4-Tetrafluorethoxyphenyl | |
| 68 | $C(CH_3)C_2H_5$ | 4-Trifluormethoxyphenyl | |
| 69 | $C(CH_3)C_2H_5$ | 4-Methoxyiminocarbyl-phenyl | |

Tabelle 1 (Fortsetzung)

| Bsp.Nr. | A | Ar | Phys. Daten |
|---|---|---|---|
| 70 | $C(CH_3)C_2H_5$ | 4-Ethoxyiminocarbyl-phenyl | |
| 71 | $C(CH_3)C_2H_5$ | 4-Methoxy-3-methylphenyl | |
| 72 | $C(CH_3)C_2H_5$ | 3-Phenoxyphenyl | |
| 73 | $C(CH_3)C_2H_5$ | 4-Phenoxyphenyl | |
| 74 | $C(CH_3)C_2H_5$ | 2-Chlor-4-(4'-chlor-phenoxy)phenyl | |
| 75 | $C(CH_3)C_2H_5$ | 4-(4'-Chlorphenoxy)-phenyl) | |
| 76 | $C(CH_3)C_2H_5$ | 3-(4'-Fluorphenoxy)-phenyl | |
| 77 | $C(CH_3)C_2H_5$ | 4-Benzyloxyphenyl | |
| 78 | $C(C_2H_5)_2$ | Phenyl | Fp. 130-132 |
| 79 | $C(C_2H_5)_2$ | 2-Methylphenyl | Fp. 92- 94 |
| 80 | $C(C_2H_5)_2$ | 3-Methylphenyl | |
| 81 | $C(C_2H_5)_2$ | 4-Methylphenyl | Fp. 87- 88 |
| 82 | $C(C_2H_5)_2$ | 2,3-Dimethylphenyl | |
| 83 | $C(C_2H_5)_2$ | 2,4-Dimethylphenyl | |
| 84 | $C(C_2H_5)_2$ | 2,5-Dimethylphenyl | |
| 85 | $C(C_2H_5)_2$ | 2,6-Dimethylphenyl | |
| 86 | $C(C_2H_5)_2$ | 3,4-Dimethylphenyl | |
| 87 | $C(C_2H_5)_2$ | 3,5-Dimethylphenyl | |
| 88 | $C(C_2H_5)_2$ | 2,4,5-Trimethylphenyl | |
| 89 | $C(C_2H_5)_2$ | 2,4,6-Trimethylphenyl | |
| 90 | $C(C_2H_5)_2$ | 2,3,5,6-Tetramethylphenyl | |
| 91 | $C(C_2H_5)_2$ | 4-Ethylphenyl | |

Tabelle 1 (Fortsetzung)

| Bsp.Nr. | A | Ar | Phys. Daten |
|---|---|---|---|
| 92 | $C(C_2H_5)_2$ | 4-Isopropylphenyl | |
| 93 | $C(C_2H_5)_2$ | 4-tert.Butylphenyl | Fp. 122-124 |
| 94 | $C(C_2H_5)_2$ | 5-Indanyl | |
| 95 | $C(C_2H_5)_2$ | 1-Naphthyl | Fp. 112-113 |
| 96 | $C(C_2H_5)_2$ | 2-Naphthyl | |
| 97 | $C(C_2H_5)_2$ | 2-Methyl-1-naphthyl | |
| 98 | $C(C_2H_5)_2$ | 4-Biphenyl | |
| 99 | $C(C_2H_5)_2$ | 2-Fluorphenyl | |
| 100 | $C(C_2H_5)_2$ | 3-Fluorphenyl | Fp. 120-122 |
| 101 | $C(C_2H_5)_2$ | 4-Fluorphenyl | Fp. 148-150 |
| 102 | $C(C_2H_5)_2$ | 2,3-Difluorphenyl | |
| 103 | $C(C_2H_5)_2$ | 2,4-Difluorphenyl | |
| 104 | $C(C_2H_5)_2$ | 2,5-Difluorphenyl | |
| 105 | $C(C_2H_5)_2$ | 2,6-Difluorphenyl | |
| 106 | $C(C_2H_5)_2$ | 3,4-Difluorphenyl | |
| 107 | $C(C_2H_5)_2$ | 3,5-Difluorphenyl | |
| 108 | $C(C_2H_5)_2$ | 2-Fluor-3-methylphenyl | |
| 109 | $C(C_2H_5)_2$ | 2,3,5,6-Tetrafluorphenyl | |
| 110 | $C(C_2H_5)_2$ | Pentafluorphenyl | |
| 111 | $C(C_2H_5)_2$ | 2-Chlorphenyl | Fp. 96-98 |
| 112 | $C(C_2H_5)_2$ | 3-Chlorphenyl | |
| 113 | $C(C_2H_5)_2$ | 4-Chlorphenyl | Fp. 128-129 |
| 114 | $C(C_2H_5)_2$ | 2,3-Dichlorphenyl | |
| 115 | $C(C_2H_5)_2$ | 2,4-Dichlorphenyl | Fp. 139-140 |
| 116 | $C(C_2H_5)_2$ | 2,5-Dichlorphenyl | |

Tabelle 1 (Fortsetzung)

| Bsp.Nr. | A | Ar | Phys. Daten |
|---|---|---|---|
| 117 | $C(C_2H_5)_2$ | 2,6-Dichlorphenyl | |
| 118 | $C(C_2H_5)_2$ | 3,4-Dichlorphenyl | |
| 119 | $C(C_2H_5)_2$ | 3,5-Dichlorphenyl | |
| 120 | $C(C_2H_5)_2$ | 2,4,5-Trichlorphenyl | |
| 121 | $C(C_2H_5)_2$ | 2,4,6-Trichlorphenyl | |
| 122 | $C(C_2H_5)_2$ | 2,3,6-Trichlorphenyl | |
| 123 | $C(C_2H_5)_2$ | 2-Chlor-5-methylphenyl | |
| 124 | $C(C_2H_5)_2$ | 3-Chlor-4-methylphenyl | |
| 125 | $C(C_2H_5)_2$ | 2-Chlor-4-fluorphenyl | |
| 126 | $C(C_2H_5)_2$ | 2-Chlor-6-fluorphenyl | |
| 127 | $C(C_2H_5)_2$ | 3-Chlor-2-fluorphenyl | |
| 128 | $C(C_2H_5)_2$ | Pentachlorphenyl | |
| 129 | $C(C_2H_5)_2$ | 2-Bromphenyl | |
| 130 | $C(C_2H_5)_2$ | 3-Bromphenyl | |
| 131 | $C(C_2H_5)_2$ | 4-Bromphenyl | |
| 132 | $C(C_2H_5)_2$ | 2-Fluor-4-bromphenyl | |
| 133 | $C(C_2H_5)_2$ | 2-Trifluormethylphenyl | |
| 134 | $C(C_2H_5)_2$ | 3-Trifluormethylphenyl | |
| 135 | $C(C_2H_5)_2$ | 4-Trifluormethylphenyl | |
| 136 | $C(C_2H_5)_2$ | 2,4-Bis(trifluormethyl)phenyl | |
| 137 | $C(C_2H_5)_2$ | 3,5-Bis(trifluormethyl)phenyl | |
| 138 | $C(C_2H_5)_2$ | 2-Methoxyphenyl | |
| 139 | $C(C_2H_5)_2$ | 3-Methoxyphenyl | |
| 140 | $C(C_2H_5)_2$ | 4-Methoxyphenyl | |
| 141 | $C(C_2H_5)_2$ | 3,4-Dimethoxyphenyl | |

Tabelle 1 (Fortsetzung)

| Bsp.Nr. | A | Ar | Phys. Daten |
|---|---|---|---|
| 142 | $C(C_2H_5)_2$ | 3,4,5-Trimethoxyphenyl | |
| 143 | $C(C_2H_5)_2$ | 4-Ethoxyphenyl | |
| 144 | $C(C_2H_5)_2$ | 4-Tetrafluorethoxyphenyl | |
| 145 | $C(C_2H_5)_2$ | 4-Trifluormethoxyphenyl | |
| 146 | $C(C_2H_5)_2$ | 4-Methoxyiminocarbyl-phenyl | |
| 147 | $C(C_2H_5)_2$ | 4-Ethoxyiminocarbyl-phenyl | |
| 148 | $C(C_2H_5)_2$ | 4-Methoxy-3-methylphenyl | |
| 149 | $C(C_2H_5)_2$ | 3-Phenoxyphenyl | |
| 150 | $C(C_2H_5)_2$ | 4-Phenoxyphenyl | |
| 151 | $C(C_2H_5)_2$ | 2-Chlor-4-(4'-chlor-phenoxy)phenyl | |
| 152 | $C(C_2H_5)_2$ | 4-(4'-Chlorphenoxy)-phenyl) | |
| 153 | $C(C_2H_5)_2$ | 4-Benzyloxyphenyl | |
| 154 | $C(CH_2)_4$ | Phenyl | |
| 155 | $C(CH_2)_4$ | 2-Methylphenyl | |
| 156 | $C(CH_2)_4$ | 3-Methylphenyl | |
| 157 | $C(CH_2)_4$ | 4-Methylphenyl | |
| 158 | $C(CH_2)_4$ | 2,3-Dimethylphenyl | |
| 159 | $C(CH_2)_4$ | 2,4-Dimethylphenyl | |
| 160 | $C(CH_2)_4$ | 2,5-Dimethylphenyl | |
| 161 | $C(CH_2)_4$ | 2,6-Dimethylphenyl | |
| 162 | $C(CH_2)_4$ | 3,4-Dimethylphenyl | |
| 163 | $C(CH_2)_4$ | 3,5-Dimethylphenyl | |

Tabelle 1 (Fortsetzung)

| Bsp.Nr. | A | Ar | Phys. Daten |
|---|---|---|---|
| 164 | $C(CH_2)_4$ | 2,4,5-Trimethylphenyl | |
| 165 | $C(CH_2)_4$ | 2,4,6-Trimethylphenyl | |
| 166 | $C(CH_2)_4$ | 2,3,5,6-Tetramethylphenyl | |
| 167 | $C(CH_2)_4$ | 4-Ethylphenyl | |
| 168 | $C(CH_2)_4$ | 4-Isopropylphenyl | |
| 169 | $C(CH_2)_4$ | 4-tert.Butylphenyl | |
| 170 | $C(CH_2)_4$ | 5-Indanyl | |
| 171 | $C(CH_2)_4$ | 1-Naphthyl | |
| 172 | $C(CH_2)_4$ | 2-Naphthyl | |
| 173 | $C(CH_2)_4$ | 2-Methyl-1-napthyl | |
| 174 | $C(CH_2)_4$ | 4-Biphenyl | |
| 175 | $C(CH_2)_4$ | 2-Fluorphenyl | Fp. 75- 76 |
| 176 | $C(CH_2)_4$ | 3-Fluorphenyl | |
| 177 | $C(CH_2)_4$ | 4-Fluorphenyl | Fp. 166-168 |
| 178 | $C(CH_2)_4$ | 2,3-Difluorphenyl | |
| 179 | $C(CH_2)_4$ | 2,4-Difluorphenyl | |
| 180 | $C(CH_2)_4$ | 2,5-Difluorphenyl | |
| 181 | $C(CH_2)_4$ | 2,6-Difluorphenyl | |
| 182 | $C(CH_2)_4$ | 3,4-Difluorphenyl | |
| 183 | $C(CH_2)_4$ | 3,5-Difluorphenyl | |
| 184 | $C(CH_2)_4$ | 2-Fluor-3-methylphenyl | |
| 185 | $C(CH_2)_4$ | 2,3,5,6-Tetrafluorphenyl | |
| 186 | $C(CH_2)_4$ | Pentafluorphenyl | |
| 187 | $C(CH_2)_4$ | 2-Chlorphenyl | Fp. 114-116 |
| 188 | $C(CH_2)_4$ | 3-Chlorphenyl | |

Tabelle 1 (Fortsetzung)

| Bsp.Nr. | A | Ar | Phys. Daten |
|---|---|---|---|
| 189 | $C(CH_2)_4$ | 4-Chlorphenyl | Fp. 162-164 |
| 190 | $C(CH_2)_4$ | 2,3-Dichlorphenyl | |
| 191 | $C(CH_2)_4$ | 2,4-Dichlorphenyl | Fp. 110-112 |
| 192 | $C(CH_2)_4$ | 2,5-Dichlorphenyl | |
| 193 | $C(CH_2)_4$ | 2,6-Dichlorphenyl | |
| 194 | $C(CH_2)_4$ | 3,4-Dichlorphenyl | |
| 195 | $C(CH_2)_4$ | 3,5-Dichlorphenyl | |
| 196 | $C(CH_2)_4$ | 2,4,5-Trichlorphenyl | |
| 197 | $C(CH_2)_4$ | 2,4,6-Trichlorphenyl | |
| 198 | $C(CH_2)_4$ | 2,3,6-Trichlorphenyl | |
| 199 | $C(CH_2)_4$ | 2-Chlor-5-methylphenyl | |
| 200 | $C(CH_2)_4$ | 3-Chlor-4-methylphenyl | |
| 201 | $C(CH_2)_4$ | 2-Chlor-4-fluorphenyl | |
| 202 | $C(CH_2)_4$ | 2-Chlor-6-fluorphenyl | |
| 203 | $C(CH_2)_4$ | 3-Chlor-2-fluorphenyl | |
| 204 | $C(CH_2)_4$ | Pentachlorphenyl | |
| 205 | $C(CH_2)_4$ | 2-Bromphenyl | |
| 206 | $C(CH_2)_4$ | 3-Bromphenyl | |
| 207 | $C(CH_2)_4$ | 4-Bromphenyl | |
| 208 | $C(CH_2)_4$ | 2-Fluor-4-bromphenyl | |
| 209 | $C(CH_2)_4$ | 2-Trifluormethylphenyl | |
| 210 | $C(CH_2)_4$ | 3-Trifluormethylphenyl | |
| 211 | $C(CH_2)_4$ | 4-Trifluormethylphenyl | |
| 212 | $C(CH_2)_4$ | 2,4-Bis(trifluormethyl)phenyl | |
| 213 | $C(CH_2)_4$ | 3,5-Bis(trifluormethyl)phenyl | |

Tabelle 1 (Fortsetzung)

| Bsp.Nr. | A | Ar | Phys. Daten |
|---|---|---|---|
| 214 | $C(CH_2)_4$ | 2-Methoxyphenyl | |
| 215 | $C(CH_2)_4$ | 3-Methoxyphenyl | |
| 216 | $C(CH_2)_4$ | 4-Methoxyphenyl | |
| 217 | $C(CH_2)_4$ | 3,4-Dimethoxyphenyl | |
| 218 | $C(CH_2)_4$ | 3,4,5-Trimethoxyphenyl | |
| 219 | $C(CH_2)_4$ | 4-Ethoxyphenyl | |
| 220 | $C(CH_2)_4$ | 4-Tetrafluorethoxyphenyl | |
| 221 | $C(CH_2)_4$ | 4-Trifluormethoxyphenyl | |
| 222 | $C(CH_2)_4$ | 4-Methoxyiminocarbyl-phenyl | |
| 223 | $C(CH_2)_4$ | 4-Ethoxyiminocarbyl-phenyl | |
| 224 | $C(CH_2)_4$ | 4-Methoxy-3-methylphenyl | |
| 225 | $C(CH_2)_4$ | 3-Phenoxyphenyl | |
| 226 | $C(CH_2)_4$ | 4-Phenoxyphenyl | |
| 227 | $C(CH_2)_4$ | 2-Chlor-4-(4'-chlor-phenoxy)phenyl | |
| 228 | $C(CH_2)_4$ | 3-(4'-Fluorphenoxy)-phenyl | |
| 229 | $C(CH_2)_4$ | 4-Benzyloxyphenyl | |
| 230 | $C(CH_2)_5$ | Phenyl | Fp. 117-119 |
| 231 | $C(CH_2)_5$ | 2-Methylphenyl | |
| 232 | $C(CH_2)_5$ | 3-Methylphenyl | |
| 233 | $C(CH_2)_5$ | 4-Methylphenyl | |
| 234 | $C(CH_2)_5$ | 2,3-Dimethylphenyl | |
| 235 | $C(CH_2)_5$ | 2,4-Dimethylphenyl | |
| 236 | $C(CH_2)_5$ | 2,5-Dimethylphenyl | |

Tabelle 1 (Fortsetzung)

| Bsp.Nr. | A | Ar | Phys. Daten |
|---|---|---|---|
| 237 | $C(CH_2)_5$ | 2,6-Dimethylphenyl | |
| 238 | $C(CH_2)_5$ | 3,4-Dimethylphenyl | |
| 239 | $C(CH_2)_5$ | 3,5-Dimethylphenyl | |
| 240 | $C(CH_2)_5$ | 2,4,5-Trimethylphenyl | |
| 241 | $C(CH_2)_5$ | 2,4,6-Trimethylphenyl | |
| 242 | $C(CH_2)_5$ | 2,3,5,6-Tetramethylphenyl | |
| 243 | $C(CH_2)_5$ | 4-Ethylphenyl | |
| 244 | $C(CH_2)_5$ | 4-Isopropylphenyl | |
| 245 | $C(CH_2)_5$ | 4-tert.Butylphenyl | |
| 246 | $C(CH_2)_5$ | 5-Indanyl | |
| 247 | $C(CH_2)_5$ | 1-Naphthyl | |
| 248 | $C(CH_2)_5$ | 2-Naphthyl | |
| 249 | $C(CH_2)_5$ | 2-Methyl-1-napthyl | |
| 250 | $C(CH_2)_5$ | 4-Biphenyl | |
| 251 | $C(CH_2)_5$ | 2-Fluorphenyl | Fp. 146-148 |
| 252 | $C(CH_2)_5$ | 3-Fluorphenyl | Fp. 132-134 |
| 253 | $C(CH_2)_5$ | 4-Fluorphenyl | Fp. 126-127 |
| 254 | $C(CH_2)_5$ | 2,3-Difluorphenyl | |
| 255 | $C(CH_2)_5$ | 2,4-Difluorphenyl | |
| 256 | $C(CH_2)_5$ | 2,5-Difluorphenyl | |
| 257 | $C(CH_2)_5$ | 2,6-Difluorphenyl | |
| 258 | $C(CH_2)_5$ | 3,4-Difluorphenyl | |
| 259 | $C(CH_2)_5$ | 3,5-Difluorphenyl | |
| 260 | $C(CH_2)_5$ | 2-Fluor-3-methylphenyl | |
| 261 | $C(CH_2)_5$ | 2,3,5,6-Tetrafluorphenyl | |

Tabelle 1 (Fortsetzung)

| Bsp.Nr. | A | Ar | Phys. Daten |
|---|---|---|---|
| 262 | $C(CH_2)_5$ | Pentafluorphenyl | |
| 263 | $C(CH_2)_5$ | 2-Chlorphenyl | Fp. 162-164 |
| 264 | $C(CH_2)_5$ | 3-Chlorphenyl | |
| 265 | $C(CH_2)_5$ | 4-Chlorphenyl | Fp. 155-156 |
| 266 | $C(CH_2)_5$ | 2,3-Dichlorphenyl | |
| 267 | $C(CH_2)_5$ | 2,4-Dichlorphenyl | Öl;N-Oxid Fp. 205-207 |
| 268 | $C(CH_2)_5$ | 2,5-Dichlorphenyl | |
| 269 | $C(CH_2)_5$ | 2,6-Dichlorphenyl | |
| 270 | $C(CH_2)_5$ | 3,4-Dichlorphenyl | Fp. 181-183 |
| 271 | $C(CH_2)_5$ | 3,5-Dichlorphenyl | |
| 272 | $C(CH_2)_5$ | 2,4,5-Trichlorphenyl | |
| 273 | $C(CH_2)_5$ | 2,4,6-Trichlorphenyl | |
| 274 | $C(CH_2)_5$ | 2,3,6-Trichlorphenyl | |
| 275 | $C(CH_2)_5$ | 2-Chlor-5-methylphenyl | |
| 276 | $C(CH_2)_5$ | 3-Chlor-4-methylphenyl | |
| 277 | $C(CH_2)_5$ | 2-Chlor-4-fluorphenyl | |
| 278 | $C(CH_2)_5$ | 2-Chlor-6-fluorphenyl | |
| 279 | $C(CH_2)_5$ | 3-Chlor-2-fluorphenyl | |
| 280 | $C(CH_2)_5$ | Pentachlorphenyl | |
| 281 | $C(CH_2)_5$ | 2-Bromphenyl | |
| 282 | $C(CH_2)_5$ | 3-Bromphenyl | |
| 283 | $C(CH_2)_5$ | 4-Bromphenyl | Fp. 190-191 |
| 284 | $C(CH_2)_5$ | 2-Fluor-4-bromphenyl | |
| 285 | $C(CH_2)_5$ | 2-Trifluormethylphenyl | |

Tabelle 1 (Fortsetzung)

| Bsp.Nr. | A | Ar | Phys. Daten |
|---|---|---|---|
| 286 | $C(CH_2)_5$ | 3-Trifluormethylphenyl | |
| 287 | $C(CH_2)_5$ | 4-Trifluormethylphenyl | |
| 288 | $C(CH_2)_5$ | 2,4-Bis(trifluormethyl)phenyl | |
| 289 | $C(CH_2)_5$ | 3,5-Bis(trifluormethyl)phenyl | |
| 290 | $C(CH_2)_5$ | 2-Methoxyphenyl | |
| 291 | $C(CH_2)_5$ | 3-Methoxyphenyl | |
| 292 | $C(CH_2)_5$ | 4-Methoxyphenyl | |
| 293 | $C(CH_2)_5$ | 3,4-Dimethoxyphenyl | |
| 294 | $C(CH_2)_5$ | 3,4,5-Trimethoxyphenyl | |
| 295 | $C(CH_2)_5$ | 4-Ethoxyphenyl | |
| 296 | $C(CH_2)_5$ | 4-Tetrafluorethoxyphenyl | |
| 297 | $C(CH_2)_5$ | 4-Trifluormethoxyphenyl | |
| 298 | $C(CH_2)_5$ | 4-Methoxyiminocarbyl-phenyl | |
| 299 | $C(CH_2)_5$ | 4-Ethoxyiminocarbyl-phenyl | |
| 300 | $C(CH_2)_5$ | 4-Methoxy-3-methylphenyl | |
| 301 | $C(CH_2)_5$ | 3-Phenoxyphenyl | |
| 302 | $C(CH_2)_5$ | 4-Phenoxyphenyl | |
| 303 | $C(CH_2)_5$ | 2-Chlor-4-(4'-chlor-phenoxy)phenyl | |
| 304 | $C(CH_2)_5$ | 3-(4'-Fluorphenoxy)-phenyl | |
| 305 | $C(CH_2)_5$ | 4-Benzyloxyphenyl | |
| 306 | $C(CH_3)nC_3H_7$ | Phenyl | Öl; N-Oxid: Fp.154-156 |
| 307 | $C(CH_3)nC_3H_7$ | 2-Methylphenyl | |

Tabelle 1 (Fortsetzung)

| Bsp.Nr. | A | Ar | Phys. Daten |
|---|---|---|---|
| 308 | $C(CH_3)nC_3H_7$ | 3-Methylphenyl | |
| 309 | $C(CH_3)nC_3H_7$ | 4-Methylphenyl | |
| 310 | $C(CH_3)nC_3H_7$ | 2,3-Dimethylphenyl | |
| 311 | $C(CH_3)nC_3H_7$ | 2,4-Dimethylphenyl | |
| 312 | $C(CH_3)nC_3H_7$ | 2,5-Dimethylphenyl | |
| 313 | $C(CH_3)nC_3H_7$ | 2,6-Dimethylphenyl | |
| 314 | $C(CH_3)nC_3H_7$ | 3,4-Dimethylphenyl | |
| 315 | $C(CH_3)nC_3H_7$ | 3,5-Dimethylphenyl | |
| 316 | $C(CH_3)nC_3H_7$ | 2,4,5-Trimethylphenyl | |
| 317 | $C(CH_3)nC_3H_7$ | 2,4,6-Trimethylphenyl | |
| 318 | $C(CH_3)nC_3H_7$ | 2,3,5,6-Tetramethylphenyl | |
| 319 | $C(CH_3)nC_3H_7$ | 4-Ethylphenyl | |
| 320 | $C(CH_3)nC_3H_7$ | 4-Isopropylphenyl | |
| 321 | $C(CH_3)nC_3H_7$ | 4-tert.Butylphenyl | |
| 322 | $C(CH_3)nC_3H_7$ | 5-Indanyl | |
| 323 | $C(CH_3)nC_3H_7$ | 1-Naphthyl | |
| 324 | $C(CH_3)nC_3H_7$ | 2-Naphthyl | |
| 325 | $C(CH_3)nC_3H_7$ | 2-Methyl-1-naphthyl | |
| 326 | $C(CH_3)nC_3H_7$ | 4-Biphenyl | |
| 327 | $C(CH_3)nC_3H_7$ | 2-Fluorphenyl | |
| 328 | $C(CH_3)nC_3H_7$ | 3-Fluorphenyl | |
| 329 | $C(CH_3)nC_3H_7$ | 4-Fluorphenyl | |
| 330 | $C(CH_3)nC_3H_7$ | 2,3-Difluorphenyl | |
| 331 | $C(CH_3)nC_3H_7$ | 2,4-Difluorphenyl | |
| 332 | $C(CH_3)nC_3H_7$ | 2,5-Difluorphenyl | |

Tabelle 1 (Fortsetzung)

| Bsp.Nr. | A | Ar | Phys. Daten |
|---|---|---|---|
| 333 | $C(CH_3)nC_3H_7$ | 2,6-Difluorphenyl | |
| 334 | $C(CH_3)nC_3H_7$ | 3,4-Difluorphenyl | |
| 335 | $C(CH_3)nC_3H_7$ | 3,5-Difluorphenyl | |
| 336 | $C(CH_3)nC_3H_7$ | 2-Fluor-3-methylphenyl | |
| 337 | $C(CH_3)nC_3H_7$ | 2,3,5,6-Tetrafluorphenyl | |
| 338 | $C(CH_3)nC_3H_7$ | Pentafluorphenyl | |
| 339 | $C(CH_3)nC_3H_7$ | 2-Chlorphenyl | |
| 340 | $C(CH_3)nC_3H_7$ | 3-Chlorphenyl | |
| 341 | $C(CH_3)nC_3H_7$ | 4-Chlorphenyl | Öl; N-Oxid: Fp. 159-160 |
| 342 | $C(CH_3)nC_3H_7$ | 2,3-Dichlorphenyl | |
| 343 | $C(CH_3)nC_3H_7$ | 2,4-Dichlorphenyl | |
| 344 | $C(CH_3)nC_3H_7$ | 2,5-Dichlorphenyl | |
| 345 | $C(CH_3)nC_3H_7$ | 2,6-Dichlorphenyl | |
| 346 | $C(CH_3)nC_3H_7$ | 3,4-Dichlorphenyl | |
| 347 | $C(CH_3)nC_3H_7$ | 3,5-Dichlorphenyl | |
| 348 | $C(CH_3)nC_3H_7$ | 2,4,5-Trichlorphenyl | |
| 349 | $C(CH_3)nC_3H_7$ | 2,4,6-Trichlorphenyl | |
| 350 | $C(CH_3)nC_3H_7$ | 2,3,6-Trichlorphenyl | |
| 351 | $C(CH_3)nC_3H_7$ | 2-Chlor-5-methylphenyl | |
| 352 | $C(CH_3)nC_3H_7$ | 3-Chlor-4-methylphenyl | |
| 353 | $C(CH_3)nC_3H_7$ | 2-Chlor-4-fluorphenyl | |
| 354 | $C(CH_3)nC_3H_7$ | 2-Chlor-6-fluorphenyl | |
| 355 | $C(CH_3)nC_3H_7$ | 3-Chlor-2-fluorphenyl | |
| 356 | $C(CH_3)nC_3H_7$ | Pentachlorphenyl | |

Tabelle 1 (Fortsetzung)

| Bsp.Nr. | A | Ar | Phys. Daten |
|---|---|---|---|
| 357 | $C(CH_3)nC_3H_7$ | 2-Bromphenyl | |
| 358 | $C(CH_3)nC_3H_7$ | 3-Bromphenyl | |
| 359 | $C(CH_3)nC_3H_7$ | 4-Bromphenyl | |
| 360 | $C(CH_3)nC_3H_7$ | 2-Fluor-4-bromphenyl | |
| 361 | $C(CH_3)nC_3H_7$ | 2-Trifluormethylphenyl | |
| 362 | $C(CH_3)nC_3H_7$ | 3-Trifluormethylphenyl | |
| 363 | $C(CH_3)nC_3H_7$ | 4-Trifluormethylphenyl | |
| 364 | $C(CH_3)nC_3H_7$ | 2,4-Bis(trifluormethyl)phenyl | |
| 365 | $C(CH_3)nC_3H_7$ | 3,5-Bis(trifluormethyl)phenyl | |
| 366 | $C(CH_3)nC_3H_7$ | 2-Methoxyphenyl | |
| 367 | $C(CH_3)nC_3H_7$ | 3-Methoxyphenyl | |
| 368 | $C(CH_3)nC_3H_7$ | 4-Methoxyphenyl | |
| 369 | $C(CH_3)nC_3H_7$ | 3,4-Dimethoxyphenyl | |
| 370 | $C(CH_3)nC_3H_7$ | 3,4,5-Trimethoxyphenyl | |
| 371 | $C(CH_3)nC_3H_7$ | 4-Ethoxyphenyl | |
| 372 | $C(CH_3)nC_3H_7$ | 4-Tetrafluorethoxyphenyl | |
| 373 | $C(CH_3)nC_3H_7$ | 4-Trifluormethoxyphenyl | |
| 374 | $C(CH_3)nC_3H_7$ | 4-Methoxyiminocarbyl-phenyl | |
| 375 | $C(CH_3)nC_3H_7$ | 4-Ethoxyiminocarbyl-phenyl | |
| 376 | $C(CH_3)nC_3H_7$ | 4-Methoxy-3-methylphenyl | |
| 377 | $C(CH_3)nC_3H_7$ | 3-Phenoxyphenyl | |
| 378 | $C(CH_3)nC_3H_7$ | 4-Phenoxyphenyl | |
| 379 | $C(CH_3)nC_3H_7$ | 2-Chlor-4-(4'-chlor-phenoxy)phenyl | |

Tabelle 1 (Fortsetzung)

| Bsp.Nr. | A | Ar | Phys. Daten |
|---|---|---|---|
| 380 | $C(CH_3)nC_3H_7$ | 3-(4'-Fluorphenoxy)-phenyl | |
| 381 | $C(CH_3)nC_3H_7$ | 4-Benzyloxyphenyl | |
| 382 | $C(C_2H_5)nC_4H_9$ | Phenyl | Öl |
| 383 | $C(C_2H_5)nC_4H_9$ | 2-Methylphenyl | |
| 384 | $C(C_2H_5)nC_4H_9$ | 3-Methylphenyl | |
| 385 | $C(C_2H_5)nC_4H_9$ | 4-Methylphenyl | |
| 386 | $C(C_2H_5)nC_4H_9$ | 2,3-Dimethylphenyl | |
| 387 | $C(C_2H_5)nC_4H_9$ | 2,4-Dimethylphenyl | |
| 388 | $C(C_2H_5)nC_4H_9$ | 2,5-Dimethylphenyl | |
| 389 | $C(C_2H_5)nC_4H_9$ | 2,6-Dimethylphenyl | |
| 390 | $C(C_2H_5)nC_4H_9$ | 3,4-Dimethylphenyl | |
| 391 | $C(C_2H_5)nC_4H_9$ | 3,5-Dimethylphenyl | |
| 392 | $C(C_2H_5)nC_4H_9$ | 2,4,5-Trimethylphenyl | |
| 393 | $C(C_2H_5)nC_4H_9$ | 2,4,6-Trimethylphenyl | |
| 394 | $C(C_2H_5)nC_4H_9$ | 2,3,5,6-Tetramethylphenyl | |
| 395 | $C(C_2H_5)nC_4H_9$ | 4-Ethylphenyl | |
| 396 | $C(C_2H_5)nC_4H_9$ | 4-Isopropylphenyl | |
| 397 | $C(C_2H_5)nC_4H_9$ | 4-tert.Butylphenyl | |
| 398 | $C(C_2H_5)nC_4H_9$ | 5-Indanyl | |
| 399 | $C(C_2H_5)nC_4H_9$ | 1-Naphthyl | |
| 400 | $C(C_2H_5)nC_4H_9$ | 2-Naphthyl | |
| 401 | $C(C_2H_5)nC_4H_9$ | 2-Methyl-1-napthyl | |
| 402 | $C(C_2H_5)nC_4H_9$ | 4-Biphenyl | |
| 403 | $C(C_2H_5)nC_4H_9$ | 2-Fluorphenyl | |

Tabelle 1 (Fortsetzung)

| Bsp.Nr. | A | Ar | Phys. Daten |
|---|---|---|---|
| 404 | $C(C_2H_5)nC_4H_9$ | 3-Fluorphenyl | |
| 405 | $C(C_2H_5)nC_4H_9$ | 4-Fluorphenyl | |
| 406 | $C(C_2H_5)nC_4H_9$ | 2,3-Difluorphenyl | |
| 407 | $C(C_2H_5)nC_4H_9$ | 2,4-Difluorphenyl | |
| 408 | $C(C_2H_5)nC_4H_9$ | 2,5-Difluorphenyl | |
| 409 | $C(C_2H_5)nC_4H_9$ | 2,6-Difluorphenyl | |
| 410 | $C(C_2H_5)nC_4H_9$ | 3,4-Difluorphenyl | |
| 411 | $C(C_2H_5)nC_4H_9$ | 3,5-Difluorphenyl | |
| 412 | $C(C_2H_5)nC_4H_9$ | 2-Fluor-3-methylphenyl | |
| 413 | $C(C_2H_5)nC_4H_9$ | 2,3,5,6-Tetrafluorphenyl | |
| 414 | $C(C_2H_5)nC_4H_9$ | Pentafluorphenyl | |
| 415 | $C(C_2H_5)nC_4H_9$ | 2-Chlorphenyl | |
| 416 | $C(C_2H_5)nC_4H_9$ | 3-Chlorphenyl | |
| 417 | $C(C_2H_5)nC^4H_9$ | 4-Chlorphenyl | |
| 418 | $C(C_2H_5)nC_4H_9$ | 2,3-Dichlorphenyl | |
| 419 | $C(C_2H_5)nC_4H_9$ | 2,4-Dichlorphenyl | |
| 420 | $C(C_2H_5)nC_4H_9$ | 2,5-Dichlorphenyl | |
| 421 | $C(C_2H_5)nC_4H_9$ | 2,6-Dichlorphenyl | |
| 422 | $C(C_2H_5)nC_4H_9$ | 3,4-Dichlorphenyl | |
| 423 | $C(C_2H_5)nC_4H_9$ | 3,5-Dichlorphenyl | |
| 424 | $C(C_2H_5)nC_4H_9$ | 2,4,5-Trichlorphenyl | |
| 425 | $C(C_2H_5)nC_4H_9$ | 2,4,6-Trichlorphenyl | |
| 426 | $C(C_2H_5)nC_4H_9$ | 2,3,6-Trichlorphenyl | |
| 427 | $C(C_2H_5)nC_4H_9$ | 2-Chlor-5-methylphenyl | |
| 428 | $C(C_2H_5)nC_4H_9$ | 3-Chlor-4-methylphenyl | |

Tabelle 1 (Fortsetzung)

| Bsp.Nr. | A | Ar | Phys. Daten |
|---|---|---|---|
| 429 | $C(C_2H_5)nC_4H_9$ | 2-Chlor-4-fluorphenyl | |
| 430 | $C(C_2H_5)nC_4H_9$ | 2-Chlor-6-fluorphenyl | |
| 431 | $C(C_2H_5)nC_4H_9$ | 3-Chlor-2-fluorphenyl | |
| 432 | $C(C_2H_5)nC_4H_9$ | Pentachlorphenyl | |
| 433 | $C(C_2H_5)nC_4H_9$ | 2-Bromphenyl | |
| 434 | $C(C_2H_5)nC_4H_9$ | 3-Bromphenyl | |
| 435 | $C(C_2H_5)nC_4H_9$ | 4-Bromphenyl | |
| 436 | $C(C_2H_5)nC_4H_9$ | 2-Fluor-4-bromphenyl | |
| 437 | $C(C_2H_5)nC_4H_9$ | 2-Trifluormethylphenyl | |
| 438 | $C(C_2H_5)nC_4H_9$ | 3-Trifluormethylphenyl | |
| 439 | $C(C_2H_5)nC_4H_9$ | 4-Trifluormethylphenyl | |
| 440 | $C(C_2H_5)nC_4H_9$ | 2,4-Bis(trifluormethyl)phenyl | |
| 441 | $C(C_2H_5)nC_4H_9$ | 3,5-Bis(trifluormethyl)phenyl | |
| 442 | $C(C_2H_5)nC_4H_9$ | 2-Methoxyphenyl | |
| 443 | $C(C_2H_5)nC_4H_9$ | 3-Methoxyphenyl | |
| 444 | $C(C_2H_5)nC_4H_9$ | 4-Methoxyphenyl | |
| 445 | $C(C_2H_5)nC_4H_9$ | 3,4-Dimethoxyphenyl | |
| 446 | $C(C_2H_5)nC_4H_9$ | 3,4,5-Trimethoxyphenyl | |
| 447 | $C(C_2H_5)nC_4H_9$ | 4-Ethoxyphenyl | |
| 448 | $C(C_2H_5)nC_4H_9$ | 4-Tetrafluorethoxyphenyl | |
| 449 | $C(C_2H_5)nC_4H_9$ | 4-Trifluormethoxyphenyl | |
| 450 | $C(C_2H_5)nC_4H_9$ | 4-Methoxyiminocarbyl-phenyl | |
| 451 | $C(C_2H_5)nC_4H_9$ | 4-Ethoxyiminocarbyl-phenyl | |
| 452 | $C(C_2H_5)nC_4H_9$ | 4-Methoxy-3-methylphenyl | |
| 453 | $C(C_2H_5)nC_4H_9$ | 3-Phenoxyphenyl | |

Tabelle 1 (Fortsetzung)

| Bsp.Nr. | A | Ar | Phys. Daten |
|---|---|---|---|
| 454 | $C(C_2H_5)nC_4H_9$ | 4-Phenoxyphenyl | |
| 455 | $C(C_2H_5)nC_4H_9$ | 2-Chlor-4-(4'-chlor-phenoxy)phenyl | |
| 456 | $C(C_2H_5)nC_4H_9$ | 3-(4'-Fluorphenoxy)-phenyl | |
| 457 | $C(C_2H_5)nC_4H_9$ | 4-Benzyloxyphenyl | |
| 458 | $C(CH_2)_3OCH_2$ | Phenyl | |
| 459 | $C(CH_2)_3OCH_2$ | 2-Methylphenyl | |
| 460 | $C(CH_2)_3OCH_2$ | 3-Methylphenyl | |
| 461 | $C(CH_2)_3OCH_2$ | 4-Methylphenyl | |
| 462 | $C(CH_2)_3OCH_2$ | 2,3-Dimethylphenyl | |
| 463 | $C(CH_2)_3OCH_2$ | 2,4-Dimethylphenyl | |
| 464 | $C(CH_2)_3OCH_2$ | 2,5-Dimethylphenyl | |
| 465 | $C(CH_2)_3OCH_2$ | 2,6-Dimethylphenyl | |
| 466 | $C(CH_2)_3OCH_2$ | 3,4-Dimethylphenyl | |
| 467 | $C(CH_2)_3OCH_2$ | 3,5-Dimethylphenyl | |
| 468 | $C(CH_2)_3OCH_2$ | 2,4,5-Trimethylphenyl | |
| 469 | $C(CH_2)_3OCH_2$ | 2,4,6-Trimethylphenyl | |
| 470 | $C(CH_2)_3OCH_2$ | 2,3,5,6-Tetramethylphenyl | |
| 471 | $C(CH_2)_3OCH_2$ | 4-Ethylphenyl | |
| 472 | $C(CH_2)_3OCH_2$ | 4-Isopropylphenyl | |
| 473 | $C(CH_2)_3OCH_2$ | 4-tert.Butylphenyl | |
| 474 | $C(CH_2)_3OCH_2$ | 5-Indanyl | |
| 475 | $C(CH_2)_3OCH_2$ | 1-Naphthyl | |
| 476 | $C(CH_2)_3OCH_2$ | 2-Naphthyl | |

Tabelle 1 (Fortsetzung)

| Bsp.Nr. | A | Ar | Phys. Daten |
|---|---|---|---|
| 477 | $C(CH_2)_3OCH_2$ | 2-Methyl-1-napthyl | |
| 478 | $C(CH_2)_3OCH_2$ | 4-Biphenyl | |
| 479 | $C(CH_2)_3OCH_2$ | 2-Fluorphenyl | |
| 480 | $C(CH_2)_3OCH_2$ | 3-Fluorphenyl | |
| 481 | $C(CH_2)_3OCH_2$ | 4-Fluorphenyl | |
| 482 | $C(CH_2)_3OCH_2$ | 2,3-Difluorphenyl | |
| 483 | $C(CH_2)_3OCH_2$ | 2,4-Difluorphenyl | |
| 484 | $C(CH_2)_3OCH_2$ | 2,5-Difluorphenyl | |
| 485 | $C(CH_2)_3OCH_2$ | 2,6-Difluorphenyl | |
| 486 | $C(CH_2)_3OCH_2$ | 3,4-Difluorphenyl | |
| 487 | $C(CH_2)_3OCH_2$ | 3,5-Difluorphenyl | |
| 488 | $C(CH_2)_3OCH_2$ | 2-Fluor-3-methylphenyl | |
| 489 | $C(CH_2)_3OCH_2$ | 2,3,5,6-Tetrafluorphenyl | |
| 490 | $C(CH_2)_3OCH_2$ | Pentafluorphenyl | |
| 491 | $C(CH_2)_3OCH_2$ | 2-Chlorphenyl | |
| 492 | $C(CH_2)_3OCH_2$ | 3-Chlorphenyl | |
| 493 | $C(CH_2)_3OCH_2$ | 4-Chlorphenyl | |
| 494 | $C(CH_2)_3OCH_2$ | 2,3-Dichlorphenyl | |
| 495 | $C(CH_2)_3OCH_2$ | 2,4-Dichlorphenyl | |
| 496 | $C(CH_2)_3OCH_2$ | 2,5-Dichlorphenyl | |
| 497 | $C(CH_2)_3OCH_2$ | 2,6-Dichlorphenyl | |
| 498 | $C(CH_2)_3OCH_2$ | 3,4-Dichlorphenyl | |
| 499 | $C(CH_2)_3OCH_2$ | 3,5-Dichlorphenyl | |
| 500 | $C(CH_2)_3OCH_2$ | 2,4,5-Trichlorphenyl | |
| 501 | $C(CH_2)_3OCH_2$ | 2,4,6-Trichlorphenyl | |

Tabelle 1 (Fortsetzung)

| Bsp.Nr. | A | Ar | Phys. Daten |
|---|---|---|---|
| 502 | $C(CH_2)_3OCH_2$ | 2,3,6-Trichlorphenyl | |
| 503 | $C(CH_2)_3OCH_2$ | 2-Chlor-5-methylphenyl | |
| 504 | $C(CH_2)_3OCH_2$ | 3-Chlor-4-methylphenyl | |
| 505 | $C(CH_2)_3OCH_2$ | 2-Chlor-4-fluorphenyl | |
| 506 | $C(CH_2)_3OCH_2$ | 2-Chlor-6-fluorphenyl | |
| 507 | $C(CH_2)_3OCH_2$ | 3-Chlor-2-fluorphenyl | |
| 508 | $C(CH_2)_3OCH_2$ | Pentachlorphenyl | |
| 509 | $C(CH_2)_3OCH_2$ | 2-Bromphenyl | |
| 510 | $C(CH_2)_3OCH_2$ | 3-Bromphenyl | |
| 511 | $C(CH_2)_3OCH_2$ | 4-Bromphenyl | |
| 512 | $C(CH_2)_3OCH_2$ | 2-Fluor-4-bromphenyl | |
| 513 | $C(CH_2)_3OCH_2$ | 2-Trifluormethylphenyl | |
| 514 | $C(CH_2)_3OCH_2$ | 3-Trifluormethylphenyl | |
| 515 | $C(CH_2)_3OCH_2$ | 4-Trifluormethylphenyl | |
| 516 | $C(CH_2)_3OCH_2$ | 2,4-Bis(trifluormethyl)phenyl | |
| 517 | $C(CH_2)_3OCH_2$ | 3,5-Bis(trifluormethyl)phenyl | |
| 518 | $C(CH_2)_3OCH_2$ | 2-Methoxyphenyl | |
| 519 | $C(CH_2)_3OCH_2$ | 3-Methoxyphenyl | |
| 520 | $C(CH_2)_3OCH_2$ | 4-Methoxyphenyl | |
| 521 | $C(CH_2)_3OCH_2$ | 3,4-Dimethoxyphenyl | |
| 522 | $C(CH_2)_3OCH_2$ | 3,4,5-Trimethoxyphenyl | |
| 523 | $C(CH_2)_3OCH_2$ | 4-Ethoxyphenyl | |
| 524 | $C(CH_2)_3OCH_2$ | 4-Tetrafluorethoxyphenyl | |
| 525 | $C(CH_2)_3OCH_2$ | 4-Trifluormethoxyphenyl | |
| 526 | $C(CH_2)_3OCH_2$ | 4-Methoxyiminocarbyl-phenyl | |

Tabelle 1 (Fortsetzung)

| Bsp.Nr. | A | Ar | Phys. Daten |
|---|---|---|---|
| 527 | $C(CH_2)_3OCH_2$ | 4-Ethoxyiminocarbyl-phenyl | |
| 528 | $C(CH_2)_3OCH_2$ | 4-Methoxy-3-methylphenyl | |
| 529 | $C(CH_2)_3OCH_2$ | 3-Phenoxyphenyl | |
| 530 | $C(CH_2)_3OCH_2$ | 4-Phenoxyphenyl | |
| 531 | $C(CH_2)_3OCH_2$ | 2-Chlor-4-(4'-chlor-phenoxy)phenyl | |
| 532 | $C(CH_2)_3OCH_2$ | 3-(4'-Fluorphenoxy)-phenyl | |
| 533 | $C(CH_2)_3OCH_2$ | 4-Benzyloxyphenyl | |
| 534 | $C(CH_2)_2$ | Phenyl | |
| 535 | $C(CH_2)_2$ | 2-Methylphenyl | |
| 536 | $C(CH_2)_2$ | 3-Methylphenyl | |
| 537 | $C(CH_2)_2$ | 4-Methylphenyl | |
| 538 | $C(CH_2)_2$ | 2,3-Dimethylphenyl | |
| 539 | $C(CH_2)_2$ | 2,4-Dimethylphenyl | |
| 540 | $C(CH_2)_2$ | 2,5-Dimethylphenyl | |
| 541 | $C(CH_2)_2$ | 2,6-Dimethylphenyl | |
| 542 | $C(CH_2)_2$ | 3,4-Dimethylphenyl | |
| 543 | $C(CH_2)_2$ | 3,5-Dimethylphenyl | |
| 544 | $C(CH_2)_2$ | 2,4,5-Trimethylphenyl | |
| 545 | $C(CH_2)_2$ | 2,4,6-Trimethylphenyl | |
| 546 | $C(CH_2)_2$ | 2,3,5,6-Tetramethylphenyl | |
| 547 | $C(CH_2)_2$ | 4-Ethylphenyl | |
| 548 | $C(CH_2)_2$ | 4-Isopropylphenyl | |
| 549 | $C(CH_2)_2$ | 4-tert.Butylphenyl | |

Tabelle 1 (Fortsetzung)

| Bsp.Nr. | A | Ar | Phys. Daten |
|---|---|---|---|
| 550 | $C(CH_2)_2$ | 5-Indanyl | |
| 551 | $C(CH_2)_2$ | 1-Naphthyl | |
| 552 | $C(CH_2)_2$ | 2-Naphthyl | |
| 553 | $C(CH_2)_2$ | 2-Methyl-1-napthyl | |
| 554 | $C(CH_2)_2$ | 4-Biphenyl | |
| 555 | $C(CH_2)_2$ | 2-Fluorphenyl | |
| 556 | $C(CH_2)_2$ | 3-Fluorphenyl | |
| 557 | $C(CH_2)_2$ | 4-Fluorphenyl | |
| 558 | $C(CH_2)_2$ | 2,3-Difluorphenyl | |
| 559 | $C(CH_2)_2$ | 2,4-Difluorphenyl | |
| 560 | $C(CH_2)_2$ | 2,5-Difluorphenyl | |
| 561 | $C(CH_2)_2$ | 2,6-Difluorphenyl | |
| 562 | $C(CH_2)_2$ | 3,4-Difluorphenyl | |
| 563 | $C(CH_2)_2$ | 3,5-Difluorphenyl | |
| 564 | $C(CH_2)_2$ | 2-Fluor-3-methylphenyl | |
| 565 | $C(CH_2)_2$ | 2,3,5,6-Tetrafluorphenyl | |
| 566 | $C(CH_2)_2$ | Pentafluorphenyl | |
| 567 | $C(CH_2)_2$ | 2-Chlorphenyl | |
| 568 | $C(CH_2)_2$ | 3-Chlorphenyl | |
| 569 | $C(CH_2)_2$ | 4-Chlorphenyl | |
| 570 | $C(CH_2)_2$ | 2,3-Dichlorphenyl | |
| 571 | $C(CH_2)_2$ | 2,4-Dichlorphenyl | |
| 572 | $C(CH_2)_2$ | 2,5-Dichlorphenyl | |
| 573 | $C(CH_2)_2$ | 2,6-Dichlorphenyl | |
| 574 | $C(CH_2)_2$ | 3,4-Dichlorphenyl | |

Tabelle 1 (Fortsetzung)

| Bsp.Nr. | A | Ar | Phys. Daten |
|---|---|---|---|
| 575 | $C(CH_2)_2$ | 3,5-Dichlorphenyl | |
| 576 | $C(CH_2)_2$ | 2,4,5-Trichlorphenyl | |
| 577 | $C(CH_2)_2$ | 2,4,6-Trichlorphenyl | |
| 578 | $C(CH_2)_2$ | 2,3,6-Trichlorphenyl | |
| 579 | $C(CH_2)_2$ | 2-Chlor-5-methylphenyl | |
| 580 | $C(CH_2)_2$ | 3-Chlor-4-methylphenyl | |
| 581 | $C(CH_2)_2$ | 2-Chlor-4-fluorphenyl | |
| 582 | $C(CH_2)_2$ | 2-Chlor-6-fluorphenyl | |
| 583 | $C(CH_2)_2$ | 3-Chlor-2-fluorphenyl | |
| 584 | $C(CH_2)_2$ | Pentachlorphenyl | |
| 585 | $C(CH_2)_2$ | 2-Bromphenyl | |
| 586 | $C(CH_2)_2$ | 3-Bromphenyl | |
| 587 | $C(CH_2)_2$ | 4-Bromphenyl | |
| 588 | $C(CH_2)_2$ | 2-Fluor-4-bromphenyl | |
| 589 | $C(CH_2)_2$ | 2-Trifluormethylphenyl | |
| 590 | $C(CH_2)_2$ | 3-Trifluormethylphenyl | |
| 591 | $C(CH_2)_2$ | 4-Trifluormethylphenyl | |
| 592 | $C(CH_2)_2$ | 2,4-Bis(trifluormethyl)phenyl | |
| 593 | $C(CH_2)_2$ | 3,5-Bis(trifluormethyl)phenyl | |
| 594 | $C(CH_2)_2$ | 2-Methoxyphenyl | |
| 595 | $C(CH_2)_2$ | 3-Methoxyphenyl | |
| 596 | $C(CH_2)_2$ | 4-Methoxyphenyl | |
| 597 | $C(CH_2)_2$ | 3,4-Dimethoxyphenyl | |
| 598 | $C(CH_2)_2$ | 3,4,5-Trimethoxyphenyl | |
| 599 | $C(CH_2)_2$ | 4-Ethoxyphenyl | |

Tabelle 1 (Fortsetzung)

| Bsp.Nr. | A | Ar | Phys. Daten |
|---|---|---|---|
| 600 | $C(CH_2)_2$ | 4-Tetrafluorethoxyphenyl | |
| 601 | $C(CH_2)_2$ | 4-Trifluormethoxyphenyl | |
| 602 | $C(CH_2)_2$ | 4-Methoxyiminocarbyl-phenyl | |
| 603 | $C(CH_2)_2$ | 4-Ethoxyiminocarbyl-phenyl | |
| 604 | $C(CH_2)_2$ | 4-Methoxy-3-methylphenyl | |
| 605 | $C(CH_2)_2$ | 3-Phenoxyphenyl | |
| 606 | $C(CH_2)_2$ | 4-Phenoxyphenyl | |
| 607 | $C(CH_2)_2$ | 2-Chlor-4-(4'-chlor-phenoxy)phenyl | |
| 608 | $C(CH_2)_2$ | 3-(4'-Fluorphenoxy)-phenyl | |
| 609 | $C(CH_2)_2$ | 4-Benzyloxyphenyl | |

Tabelle 2

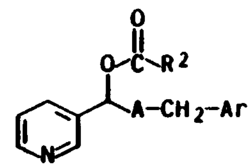

| Bsp.Nr. | $R^2$ | A | Ar | Phys. Daten |
|---|---|---|---|---|
| 610 | $CH_3$ | $C(C_2H_5)_2$ | Phenyl | Öl |
| 611 | $CH_3$ | $C(C_2H_5)_2$ | 4-Chlorphenyl | Öl |
| 612 | $CH_3$ | $C(C_2H_5)_2$ | 4-Fluorphenyl | Öl |
| 613 | $CH_3$ | $C(C_2H_5)_2$ | 2,4-Dichlorphenyl | Öl |
| 614 | $CH_3$ | $C(C_2H_5)_2$ | 2-Methylphenyl | |
| 615 | $CH_3$ | $C(C_2H_5)_2$ | 4-Methylphenyl | |
| 616 | $CH_3$ | $C(C_2H_5)_2$ | 4-tert.-Butylphenyl | |
| 617 | $CH_3$ | $C(C_2H_5)_2$ | 2-Naphthyl | |
| 618 | $CH_3$ | $C(C_2H_5)_2$ | 2-Fluorphenyl | |
| 619 | $CH_3$ | $C(C_2H_5)_2$ | 4-Bromphenyl | |
| 620 | $CH_3$ | $C(C_2H_5)_2$ | 3-Fluorphenyl | |
| 621 | $CH_3$ | $C(C_2H_5)_2$ | 3,4-Dichlorphenyl | |
| 622 | $CH_3$ | $C(CH_2)_5$ | Phenyl | |
| 623 | $CH_3$ | $C(CH_2)_5$ | 2-Methylphenyl | |
| 624 | $CH_3$ | $C(CH_2)_5$ | 4-Methylphenyl | |
| 625 | $CH_3$ | $C(CH_2)_5$ | 4-tert.-Butylphenyl | |
| 626 | $CH_3$ | $C(CH_2)_5$ | 2-Naphthyl | |
| 627 | $CH_3$ | $C(CH_2)_5$ | 2-Fluorphenyl | |

Tabelle 2 (Fortsetzung)

| Bsp.Nr. | $R^2$ | A | Ar | Phys. Daten |
|---|---|---|---|---|
| 628 | $CH_3$ | $C(CH_2)_5$ | 3-Fluorphenyl | |
| 629 | $CH_3$ | $C(CH_2)_5$ | 4-Fluorphenyl | |
| 630 | $CH_3$ | $C(CH_2)_5$ | 4-Bromphenyl | |
| 631 | $CH_3$ | $C(CH_2)_5$ | 4-Chlorphenyl | |
| 632 | $CH_3$ | $C(CH_2)_5$ | 2-Chlorphenyl | |
| 633 | $CH_3$ | $C(CH_2)_5$ | 2,4-Dichlorphenyl | |
| 634 | $CH_3$ | $C(CH_2)_5$ | 3,4-Dichlorphenyl | |
| 635 | $CH_3$ | $C(CH_2)_4$ | Phenyl | |
| 636 | $CH_3$ | $C(CH_2)_4$ | 2-Methylphenyl | |
| 637 | $CH_3$ | $C(CH_2)_4$ | 4-Methylphenyl | |
| 638 | $CH_3$ | $C(CH_2)_4$ | 4-tert.-Butylphenyl | |
| 639 | $CH_3$ | $C(CH_2)_4$ | 2-Naphthyl | |
| 640 | $CH_3$ | $C(CH_2)_4$ | 2-Fluorphenyl | |
| 641 | $CH_3$ | $C(CH_2)_4$ | 3-Fluorphenyl | |
| 642 | $CH_3$ | $C(CH_2)_4$ | 4-Fluorphenyl | |
| 643 | $CH_3$ | $C(CH_2)_4$ | 4-Bromphenyl | |
| 644 | $CH_3$ | $C(CH_2)_4$ | 4-Chlorphenyl | |
| 645 | $CH_3$ | $C(CH_2)_4$ | 2-Chlorphenyl | |
| 646 | $CH_3$ | $C(CH_2)_4$ | 2,4-Dichlorphenyl | |
| 647 | $CH_3$ | $C(CH_2)_4$ | 3,4-Dichlorphenyl | |
| 648 | $CH_3$ | $C(C_2H_5)^nC_4H_9$ | Phenyl | |
| 649 | $CH_3$ | $C(C_2H_5)^nC_4H_9$ | 2-Methylphenyl | |
| 650 | $CH_3$ | $C(C_2H_5)^nC_4H_9$ | 4-Methylphenyl | |
| 651 | $CH_3$ | $C(C_2H_5)^nC_4H_9$ | 4-tert.-Butylphenyl | |
| 652 | $CH_3$ | $C(C_2H_5)^nC_4H_9$ | 2-Naphthyl | |

Tabelle 2 (Fortsetzung)

| Bsp.Nr. | $R^2$ | A | Ar | Phys. Daten |
|---|---|---|---|---|
| 653 | $CH_3$ | $C(C_2H_5)^nC_4H_9$ | 2-Fluorphenyl | |
| 654 | $CH_3$ | $C(C_2H_5)^nC_4H_9$ | 3-Fluorphenyl | |
| 655 | $CH_3$ | $C(C_2H_5)^nC_4H_9$ | 4-Fluorphenyl | |
| 656 | $CH_3$ | $C(C_2H_5)^nC_4H_9$ | 4-Bromphenyl | |
| 657 | $CH_3$ | $C(C_2H_5)^nC_4H_9$ | 4-Chlorphenyl | |
| 658 | $CH_3$ | $C(C_2H_5)^nC_4H_9$ | 2,4-Dichlorphenyl | |
| 659 | $CH_3$ | $C(C_2H_5)^nC_4H_9$ | 3,4-Dichlorphenyl | |
| 660 | $CH_3$ | $C(C_2H_5)^nC_4H_9$ | 4-Biphenyl | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,

Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g, je Kilogramm Saatgut verwendet.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 230 (Tab. 1) mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 306 (Tab. 1) werden in einer Mischung gelöst, die aus 80 Gew.-Teilen Xylol, 10 Gew.-Teilen des Anlagerungsproduktes von 8 bis 10 Mol Ethylenoxid an 1 Mol ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 341 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 382 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 175 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 187 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 191 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 253 werden mit 10 Gew.-Teilen Natriumsalz eines Phenolsulfonsäure-harnstoff-formaldehyd-Konden sates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 267 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde 3-Phenyl-2,2-dimethyl-1-(3-pyridyl)propanol-1 (A) - bekannt aus EP-302 365 - benutzt.

Anwendungsbeispiel 1

Wirksamkeit gegen Botrytis cinerea

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten. Es wurde der Befall der Blätter geprüft.

Das Ergebnis zeigt, daß die Wirkstoffe 230, 306, 341 und 382 bei der Anwendung als 0,05 %ige Spritzbrühe eine bessere fungizide Wirkung zeigen (90 %) als der bekannte Vergleichswirkstoff A (55 %).

Anwendungsbeispiel 2

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18 °C gestellt. Anschließend wurden die Pflanzen im Gewächshaus bei 20-22 °C und 70 % relativer Luftfeuchtigkeit für weitere 5 Stunden kultiviert. Dann wurde das Ausmaß des Befalls der Blätter ermittelt.

Das Ergebnis zeigt, daß die Wirkstoffe 175, 187, 191, 253, 267, 306, 341 und 382 bei der Anwendung als 0,025 %ige Spritzbrühe eine bessere fungizide Wirkung zeigen (98 %) als der bekannte Vergleichswirkstoff A (60 %).

**Patentansprüche**

**1.** Pyridine der Formel

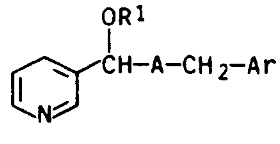

I

in der

$R^1$ für Wasserstoff, $C_1$-$C_6$-Alkyl, $C_3$-$C_6$-Alkenyl, $C_3$-$C_6$-Alkinyl oder einen Acylrest $COR^2$

steht;

$R^2$ für $C_1$-$C_6$-Alkyl, $C_3$-$C_6$-Alkenyl, $C_3$-$C_6$-Alkinyl, $C_1$-$C_6$-Halogenalkyl, Naphthyl oder Phenyl, die gegebenenfalls ein bis drei Substituenten, ausgewählt aus der Gruppe bestehend aus $C_1$-$C_4$-Alkyl, $C_1$-$C_4$-Alkoxy, $C_1$-$C_4$-Halogenalkyl, Halogen, Cyano und Nitro, tragen, steht;

A für die Gruppen

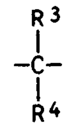 , 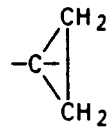 oder 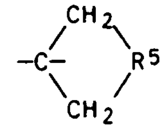

steht;

$R^3$, $R^4$ unabhängig voneinander für $C_1$-$C_6$-Alkyl stehen, mit der Maßgabe, daß nicht $R^3$ und $R^4$ gleichzeitig Methyl bedeuten;

$R^5$ für eine der Gruppen $(CH_2)_n$, $CH_2OCH_2$, $CH_2S(O)_mCH_2$, $CH_2CH_2O$, $CH_2CH_2$ $S(O)_m$ steht, wobei

n eine ganze Zahl von 1 bis 5 und

m eine ganze Zahl von 0 bis 2 bedeutet;

Ar für Phenyl, Naphthyl oder Indanyl steht, die gegebenenfalls 1-5 Substituenten aus der Gruppe $C_1$-$C_6$-Alkyl, Phenyl, Halogen, $C_1$-$C_6$-Halogenalkyl, $C_1$-$C_6$-Alkoxy, $C_1$-$C_6$-Halogenalkoxy, $C_1$-$C_6$-Alkoxyiminocarbyl, Phenoxy, Halogenphenoxy, Benzyloxy tragen, und

die N-Oxide und die pflanzenverträglichen Säureadditionssalze der Pyridinverbindungen.

**2.** Verfahren zur Herstellung eines Pyridins der Formel 1 gemäß Anspruch 1, dadurch gekennzeichnet, daß man einen Aldehyd der Formel 2,

$Ar$-$CH_2$-A-CHO 2

in der A und Ar die in Anspruch 1 genannten Bedeutungen haben, umsetzt, mit einer Organometallverbindung der Formel 3,

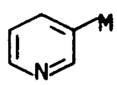 3

in der M für Lithium oder einen der Reste MgCl, MgBr, MgI steht und den dabei gebildeten Pyridylalkohol der Formel (1), in der A und Ar die in Anspruch 1 genannten Bedeutungen haben und $R^1$ für Wasserstoff steht, gegebenenfalls weiter umsetzt mit einer Verbindung der Formel 4 oder der Formel 5,

$R^1-X$

4

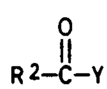

5

in denen $R^1$ und $R^2$ die in Anspruch 1 genannten Bedeutungen mit Ausnahme von Wasserstoff haben und X und Y für eine nucleofuge Gruppe stehen.

**3.** Fungizides Mittel, enthaltend einen Trägerstoff und eine fungizid wirksame Menge einer Pyridinverbindung der Formel 1 gemäß Anspruch 1.

**4.** Verfahren zur Bekämpfung von Pilzen, dadurch gekennzeichnet, daß man die Pilze oder die vom Pilzbefall bedrohten Materialien, Pflanzen, Böden oder Saatgüter behandelt mit einer fungizid wirksamen Menge einer Pyridinverbindung der Formel 1 gemäß Anspruch 1.

5. Verbindung der Formel 1 gemäß Anspruch 1, dadurch gekennzeichnet, daß $R^1$ Wasserstoff, A den Rest $C(CH_2)_5$ und Ar Phenyl bedeutet.

6. Verbindung der Formel 1 gemäß Anspruch 1, dadurch gekennzeichnet, daß $R^1$ Wasserstoff, A den Rest $C(CH_3)nC_3H_7$ und Ar Phenyl bedeutet.

7. Verbindung der Formel 1 gemäß Anspruch 1, dadurch gekennzeichnet, daß $R^1$ Wasserstoff, A den Rest $C(CH_3)nC_3H_7$ und Ar 4-Chlorphenyl bedeutet.

8. Verbindung der Formel 1 gemäß Anspruch 1, dadurch gekennzeichnet, daß $R^1$ Wasserstoff, A den Rest $C(C_2H_5)nC_4H_9$ und Ar Phenyl bedeutet.

9. Verbindung der Formel 1 gemäß Anspruch 1, dadurch gekennzeichnet, daß $R^1$ Wasserstoff, A den Rest $C(CH_2)_4$ und Ar 4-Fluorphenyl bedeutet.

## Claims

1. A pyridine of the formula

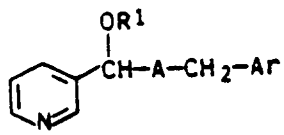

I

where

| | |
|---|---|
| $R^1$ | is hydrogen, $C_1$-$C_6$-alkyl, $C_3$-$C_6$-alkenyl, $C_3$-$C_6$-alkynyl or an acyl radical $COR^2$; |
| $R^2$ | is $C_1$-$C_6$-alkyl, $C_3$-$C_6$-alkenyl, $C_3$-$C_6$-alkynyl, $C_1$-$C_6$-haloalkyl, naphthyl or phenyl, each of which may carry from one to three substituents selected from the group consisting of $C_1$-$C_4$-alkyl, $C_1$-$C_4$-alkoxy, $C_1$-$C_4$-haloalkyl, halogen, cyano and nitro; |
| A | is |

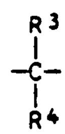 , 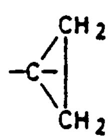 or 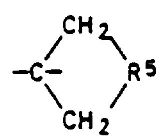

| | |
|---|---|
| $R^3$ and $R^4$, | independently of one another, are each $C_1$-$C_6$-alkyl, with the proviso that $R^3$ and $R^4$ are not both methyl; |
| $R^5$ | is $(CH_2)_n$, $CH_2OCH_2$, $CH_2S(O)_mCH_2$, $CH_2CH_2O$ or $CH_2CH_2$ $S(O)_m$, where n is an integer from 1 to 5 and m is an integer from 0 to 2; |
| Ar | is phenyl, naphthyl or indanyl, each of which may carry 1-5 substituents from the group consisting of $C_1$-$C_6$-alkyl, phenyl, halogen, $C_1$-$C_6$-haloalkyl, $C_1$-$C_6$-alkoxy, $C_1$-$C_6$-haloalkoxy, $C_1$-$C_6$-alkoxyiminomethyl, phenoxy, halophenoxy and benzyloxy, or an N-oxide or plant-tolerated acid addition salt of the pyridine. |

2. A process for the preparation of a pyridine of the formula 1 as claimed in claim 1, which comprises reacting an aldehyde of the formula 2

$Ar$-$CH_2$-A-CHO     2

in which A and Ar have the meanings specified in claim 1, with an organometallic compound of the formula 3

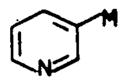

3

in which M is lithium or MgCl, MgBr or MgI, and, if appropriate, further reacting the resulting pyridyl alcohol of the formula 1 in which A and Ar have the meanings specified in claim 1, and $R^1$ is hydrogen, with a compound of the formula 4 or of the formula 5

$R^1-X$

4

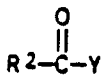

5

in which $R^1$ and $R^2$ have the meanings specified in claim 1 with the exception of hydrogen, and X and Y are each a nucleofugic group.

**3.** A fungicide containing a carrier and a fungicidally effective amount of a pyridine compound of the formula 1 as claimed in claim 1.

**4.** A method for controlling fungi, wherein the fungi, or the materials, plants, soils or seed threatened by fungal attack are treated with a fungicidally effective amount of a pyridine compound of the formula 1 as claimed in claim 1.

**5.** A compound of the formula 1 as claimed in claim 1, wherein $R^1$ is hydrogen, A is $C(CH_2)_5$ and Ar is phenyl.

**6.** A compound of the formula 1 as claimed in claim 1, wherein $R^1$ is hydrogen, A is $C(CH_3)nC_3H_7$ and Ar is phenyl.

**7.** A compound of the formula 1 as claimed in claim 1, wherein $R^1$ is hydrogen, A is $C(CH_3)nC_3H_7$ and Ar is 4-chlorophenyl.

**8.** A compound of the formula 1 as claimed in claim 1, wherein $R^1$ is hydrogen, A is $C(C_2H_5)nC_4H_9$ and Ar is phenyl.

**9.** A compound of the formula 1 as claimed in claim 1, wherein $R^1$ is hydrogen, A is $C(CH_2)_4$ and Ar is 4-fluorophenyl.

**Revendications**

**1.** Pyridines de formule

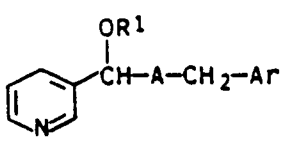

1

dans laquelle

$R^1$ est mis pour hydrogène, alkyle en C1-C6, alcényle en C3-C6, alcynyle en C3-C6 ou un reste acyle COR2;

$R^2$ est mis pour alkyle en C1-C6, alcényle en C3-C6, alcynyle en C3-C6, halogénalkyle en C1-C6, naphtyle ou phényle, qui portent éventuellement un à trois substituants choisis dans le groupe constitué d'alkyle en C1-C4, alcoxy en C1-C4, halogénalkyle en C1-C4, halogène, cyano et nitro;

A est mis pour les groupes

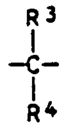 , 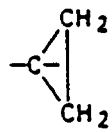 ou 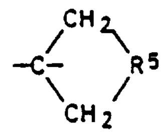 ;

$R^3$, $R^4$ indépendamment l'un de l'autre, sont mis pour alkyle en C1-C6 étant entendu que $R^3$ et $R^4$ ne représentent pas à la fois méthyle;
$R^5$ est mis pour un des groupes $(CH_2)_n$, $CH_2OCH_2$, $CH_2S(0)_mCH_2$, $CH_2CH_20$, $CH_2CH_2$ $S(0)_m$ où
n est un nombre entier allant de 1 à 5 et
m un nombre entier allant de 0 à 2;
Ar est mis pour phényle, naphtyle ou indanyle qui portent éventuellement 1 à 5 substituants choisis dans le groupe constitué d'alkyle en C1-C6, phényle, halogène, halogénalkyle en C1-C6, alcoxy en C1-C6, halogénalcoxy en C1-C6, alcoxy en C1-C6-iminocarbyle, phénoxy, halogénophénoxy, benzyloxy, et les N-oxydes et les sels d'addition d'acide des dérivés de pyridine, qui sont tolérés par les plantes.

**2.** Procédé de préparation d'une pyridine de formule 1, selon la revendication 1, caractérisé par le fait que l'on met à réagir un aldéhyde de formule 2,

$Ar-CH_2-A-CHO$ 2

dans laquelle A et Ar ont les significations données dans la revendication 1, avec un composé organométallique de formule 3

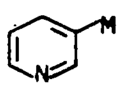 3

dans laquelle M est mis pour lithium ou un des restes MgCl, MgBr, MgI et, le cas échéant, on poursuit la réaction de l'alcool pyridique alors formé, de formule (1), où A et Ar ont les significations données dans la revendication 1 et $R^1$ est mis pour hydrogène, avec un composé de formule 4 ou de formule 5,

$R^1-X$

4

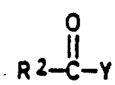

5

dans lesquelles $R^1$ et $R^2$ ont les significations données dans la revendication 1, excepté l'hydrogène, et X et Y sont mis pour un groupe nucléofuge.

**3.** Agent fongicide, contenant un support et une quantité efficace au point de vue fongicide d'un dérivé de pyridine de formule 1, selon la revendication 1.

**4.** Procédé de lutte contre les champignons, caractérisé par le fait que l'on traite les champignons ou les matériaux, plantes, sols ou semences menacés par l'attaque par les champignons avec une quantité efficace au point de vue fongicide d'un dérivé de pyridine de formule 1, selon la revendication 1.

**5.** Composé de formule 1, selon la revendication 1, caractérisé par le fait que $R^1$ représente hydrogène, A le reste $C(CH_2)_5$ et Ar phényle.

**6.** Composé de formule 1, selon la revendication 1, caractérisé par le fait que $R^1$ représente hydrogène, A le reste $C(CH_3)_nC_3H_7$ et Ar phényle.

**7.** Composé de formule 1, selon la revendication 1, caractérisé par le fait que $R^1$ représente hydrogène, A le reste $C(CH_3)_nC_3H_7$ et Ar 4-chlorophényle.

**8.** Composé de formule 1, selon la revendication 1, caractérisé par le fait que $R^1$ représente hydrogène, A le reste $C(C_2H_5)_nC_4H_9$ et Ar phényle.

**9.** Composé de formule 1, selon la revendication 1, caractérisé par le fait que $R^1$ représente hydrogène, A le reste $C(CH_2)_4$ et Ar 4-fluorophényle.